# EUROPÄISCHE PATENTSCHRIFT

(11) **EP 3 908 244 B1**
(45) Veröffentlichungstag und Bekanntmachung des Hinweises auf die Patenterteilung: **07.02.2024**
(21) Anmeldenummer: 19835604.0
(22) Anmeldetag: 10.12.2019
(51) Int. Cl.: A61J 3/00

(54) **VORRICHTUNG UND VERFAHREN FÜR DIE TABLETTIERUNG EINER PULVERFÖRMIGEN, FLÜSSIGEN, PASTÖSEN, VERKAPSELTEN ODER GRANULAREN WIRKSTOFFZUSAMMENSETZUNG**
DEVICE AND PROCESS FOR TABLETING A PULVERULENT, LIQUID, PASTY, ENCAPSULATED, OR GRANULAR ACTIVE INGREDIENT COMPOSITION
DISPOSITIF ET PROCÉDÉ DE MISE EN COMPRIMÉS D'UNE COMPOSITION DE PRINCIPES ACTIFS PULVÉRULENTE, LIQUIDE, PÂTEUSE, ENCAPSULÉE OU GRANULAIRE

(30) Priorität: 07.01.2019 DE 102019000018; 07.01.2019 DE 102019000016; 15.01.2019 DE 102019000199
(43) Veröffentlichungstag der Anmeldung: 17.11.2021
(73) Patentinhaber: LuxCan Innovation S.A., 1273 Luxembourg-Hamm (LU)
(72) Erfinder: SCHMITT, Fritz, 1855 Luxemburg (LU)
(74) Vertreter: Angerhausen, Christoph
(86) Internationale Anmeldenummer: PCT/DE2019/101067
(87) Internationale Veröffentlichungsnummer: WO 2020/143864

(56) Entgegenhaltungen:
- EP-A1- 2 532 349
- WO-A1-2017/075096
- WO-A1-2017/175057
- WO-A2-01/87272

## Beschreibung

Die Erfindung betrifft eine Vorrichtung sowie ein Verfahren für die Tablettierung einer pulverförmigen, flüssigen, pastösen, verkapselten oder granularen Wirkstoffzusammensetzung. Es werden insbesondere eine Vorrichtung sowie ein Verfahren für die Fertigung einer pulverförmigen, flüssigen, pastösen, verkapselten oder granularen einzeldosierten Wirkstoffform beschrieben, wobei mindestens ein Anteil der Zusammensetzung Wirkstoffe aus der Hanfpflanze (Cannabis) enthalten kann. Eine derartige Vorrichtung gemäß dem Oberbegriff des Anspruchs 1 sowie eine entsprechendes Verfahren sind aus der WO 01/87272 A2 bekannt. Ähnliche Gegenstände beschreiben auch die WO 2017/175057 A1 und die EP 2 532 349 A1.

Weiterhin bekannt sind Tabletten, die unter Pressdruck aus Pulvern, Granulaten oder anderen Wirkstoffvorläuferprodukten auf Tablettenpressen gefertigt werden. Tabletten können unterschiedliche Formen aufweisen. Bei Tabletten zur Einnahme ist besonders die bikonvexe Form verbreitet. Tabletten zur arzneilichen Verwendung zählen zu den Arzneimitteln in einer sonstigen gesundheitsbezogenen Verwendung und sind den Medizinprodukten oder Nahrungsergänzungsmitteln zuzurechnen. Auch in anderen Bereichen kommen Tabletten zum Einsatz. Unter den Arzneiformen nehmen Tabletten mit einem Anteil von nahezu 50 % eine besondere Rolle ein.

Große Mengen von Tabletten bleiben jährlich ungenutzt, da sie aufgrund zu großer Konfektionierung vor dem Ablauf des Haltbarkeitsdatums nicht verbraucht werden können. Es wird geschätzt, dass 30% aller verordneten Medikamente ungenutzt entsorgt werden.

Zudem werden auch heute Medikamente noch so verordnet, als würden alle Patienten identische biologische Voraussetzungen für eine Therapie mitbringen. Um bessere Behandlungserfolge zu erzielen, fordert der medizinische Fortschritt jedoch zunehmend, dass auch individuelle Merkmale der Patienten bei der Medikamentenverordnung berücksichtigt und maßgeschneiderte Therapien angeboten werden. Ebenso ist in vielen Fällen die gegenüber der Standartmedikation sparsamere Dosierung von Medikamenten hinreichend, wodurch auch das Risiko für Nebenwirkungen reduziert wird. Es besteht daher ein hoher Bedarf für die individuelle Medikation insbesondere in Tablettenform.

Aus dem Stand der Technik sind 3D-Drucker beispielsweise auch aus der Verwendung in der Lebensmitteltechnologie bekannt. Dabei wird häufig eine Lebensmittelkomponente im fließfähigen Zustand in eine gewünschte dreidimensionale Form gebracht und anschließend ausgehärtet. Solche Verfahren kommen bislang beispielsweise bei der Herstellung von Formen aus Schokolade und dergleichen zum Einsatz. So beschreibt beispielsweise die WO 2011/117012 A1 das selektive Aufbringen von Nahrungsergänzungsmitteln auf Lebensmittelprodukte. Die US 2013/0034633 A1 beschreibt ein System zur Herstellung eines frei geformten dreidimensionalen Lebensmittelprodukts. Des Weiteren wird ein Verfahren beschrieben, mit Hilfe dessen sich bestimmte Eigenschaften eines hergestellten Lebensmittelprodukts variieren lassen können.

Die EP 2 937 206 A1 beschreibt ein Verfahren für die Herstellung von Lebensmitteln auf Schokoladenbasis, bei dem die Schokolade nach dem Dosiervorgang ausgehärtet wird, um als Kristallisationsinitiator für eine folgende Schicht zu dienen. Die feste Schokolade soll eine vollständige innere und äußere dreidimensionale Struktur des Lebensmittels bilden, in die flüssige oder gasförmige Substanzen in innenliegenden Kammern der Struktur mit festen Wänden eingebaut werden können.

Die WO 2010/151302 A1 offenbart ein Verfahren für das Drucken von Lebensmitteln, bei dem kleine Tropfen eines flüssigen Lebensmittels und eines flüssigen Binders, der vorzugsweise aus einem Geliermittel besteht, das sich bei Druckbeaufschlagung verfestigt, verarbeitet werden.

Die US 2013/0034633 A1 zeigt ein Herstellungsverfahren für ein dreidimensionales Lebensmittelprodukt, bei dem in einer Pulverschicht durch lokal begrenzte Aufbringung von Flüssigkeitstropfen durch die Verwendung von Flüssigkeit und Pulver eine lokale Verfestigung herbeigeführt wird. Durch die Aufbringung weiterer Pulverschichten und Strukturen von Flüssigkeitstropfen bildet sich nach der Entfernung nicht verfestigter Pulverbestandteile eine dreidimensionale Lebensmittelstruktur.

Es ist daher die Aufgabe der Erfindung, eine Vorrichtung und ein Verfahren für die Tablettierung von Wirkstoffzusammensetzungen bereitzustellen, welche hoch flexibel an sich wechselnde Wirkstoffzusammensetzungen angepasst werden können und skalierbar sind.

Diese Aufgabe wird durch eine Vorrichtung mit den Merkmalen des Anspruchs 1 gelöst. Der nebengeordnete Anspruch 13 betrifft ein entsprechendes Verfahren. Vorteilhafte Ausführungsformen sind jeweils Gegenstand der abhängigen Ansprüche.

Demgemäß wird eine Vorrichtung für die Tablettierung einer pulverförmigen, flüssigen, pastösen, verkapselten oder granularen Wirkstoffzusammensetzung vorgeschlagen, mit mindestens einer Matrizenkammer und einem Magazin, das eine Vielzahl Wirkstoffbehälter mit jeweils einer einzeln ansteuerbaren Dosiereinrichtung und einer Düse für den Wirkstoffauslass aufweist. Das Magazin ist zwischen einer Vielzahl Stellpositionen relativ zu der mindestens einen Matrizenkammer verstellbar, wobei in jeder der Stellpositionen mindestens eine der Düsen einer Öffnung der Matrizenkammer zugewandt ist.

Das Magazin ist kugelförmig ausgebildet und sich die Wirkstoffbehälter in Radialrichtung des Magazins erstrecken sowie mit ihrer jeweiligen Düse in eine Kugeloberfläche des Magazins münden. Es kann vorgesehen sein, dass der oder die in dem Wirkstoffbehälter vorgehaltenen Wirkstoffe in dem Wirkstoffbehälter fluidisch getrennt zur Umgebung der Vorrichtung gelagert sind. Die Wirkstoffbehälter können zumindest teilweise eine Temperiereinrichtung aufweisen, um einen in einem der Wirkstoffbehälter vorgehaltenen Wirkstoff auf einer vorgegebenen Temperatur zu halten oder vor der Dosierung in die Matrizenkammer auf eine bestimmte Temperatur zu bringen, die eine chemische Umwandlung des Wirkstoffs fördert oder eine physikalische Eigenschaft des Wirkstoffs verändert.

Die mindestens eine Matrizenkammer kann in einer Grundplatte aufgenommen sein, die eine konkave Ausnehmung aufweist, in der das kugelförmige Magazin aufgenommen ist. Dabei kann an einer tiefsten Stelle der Ausnehmung die mindestens eine Matrizenkammer angeordnet sein.

Das Magazin kann um mindestens eine sich parallel zu der Grundplatte erstreckende Achse relativ zu der Grundplatte verstellbar sein.

Das kugelförmige Magazin kann in seinem Zentrum eine Kavität aufweisen, in der ein kugelförmiges Betätigungselement frei beweglich und formschlüssig oder zumindest annähernd formschlüssig aufgenommen ist. Dabei kann das Betätigungselement ein Stellglied aufweisen, das zwischen einer Betätigungsposition und einer Freigabeposition verstellbar ist. Das Betätigungselement kann einen exzentrischen Schwerpunkt aufweisen, der entlang einer linearen Verstellrichtung des Stellglieds zwischen der Betätigungsposition und der Freigabeposition angeordnet ist.

Das kugelförmige Betätigungselement kann einen sich durch sein Zentrum erstreckenden Durchlass aufweisen, in dem eine Magnetspule mit einem entlang der Spulenachse beweglichen Spulenkern angeordnet ist.

Der Durchlass kann derart in Bezug auf den Schwerpunkt des kugelförmigen Bestätigungselements angeordnet sein, dass der Durchlass unabhängig von der Ausrichtung des Magazins vertikal ausgerichtet ist.

Das Magazin kann einen linear verstellbaren Stempel aufweisen, der in einer der Stellpositionen oder in einer weiteren Stellposition des Magazins relativ zu der Matrizenkammer der Öffnung der Matrizenkammer zugewandt ist.

Der Stempel kann an einem freien Ende eine Stempelmatrize aufweisen, mit der der Stempel in einer ausgefahrenen Stellung über die Öffnung in die Matrizenkammer hineinragt und mit der der Stempel in einer Freigabeposition außer Eingriff mit der Matrizenkammer steht.

Mindestens einer der Wirkstoffbehälter kann eine Verarbeitungseinrichtung für einen Wirkstoff aufweisen. Die Verarbeitungseinrichtung kann ein Heizelement zum Erhitzen und/oder ein Kühlelement zum Kühlen eines Wirkstoffs sein oder ein solches Element aufweisen.

Das Magazin kann zusätzlich zu der Vielzahl Wirkstoffbehälter mindestens einen Trägermaterialbehälter mit einer einzeln ansteuerbaren Dosiereinrichtung und einer Düse für den Trägermaterialauslass aufweisen. Dabei kann in einer weiteren Stellposition des Magazins die Düse des Trägermaterialbehälters einer Öffnung der Matrizenkammer und/oder einer Grundplatte der Vorrichtung zugewandt sein, die die Matrize einer Tablettenverpackung aufweist.

Die Matrize kann eine Mehrzahl der Matrizenkammern aufweisen, die vorzugsweise in einem regelmäßigen Raster angeordnet sind.

Das Verfahren für die Tablettierung eines Wirkstoffs mit einer Vorrichtung der zuvor beschriebenen Art kann die Schritte aufweisen:
a) Vorgeben einer zu tablettierenden Wirkstoffzusammensetzung;
b) Verstellen des Magazins relativ zu der Matrizenkammer, bis das Magazin eine Stellposition einnimmt, in der derjenige Wirkstoffbehälter des Magazins mit seiner Düse der Matrizenkammer zugewandt ist, in dem ein Wirkstoff der Wirkstoffzusammensetzung vorgehalten ist;
c) Dosieren einer durch die Wirkstoffzusammensetzung vorgegebenen Dosis des ersten Wirkstoffs in die Matrizenkammer; und
d) optional Wiederholen der Schritte b) und c) für jeden weiteren Wirkstoff der Wirkstoffzusammensetzung, wenn die Wirkstoffzusammensetzung mehr als einen Wirkstoff aufweist.

Das Verfahren kann die folgenden weiteren Schritte aufweisen:
e) Verstellen des Magazins relativ zu der Matrizenkammer, bis das Magazin eine Stellposition einnimmt, in der ein Bindemittelvorratsbehälter des Magazins mit seiner Düse der Matrizenkammer zugewandt ist, in dem ein Bindemittel vorgehalten ist;
f) Dosieren einer durch die Wirkstoffzusammensetzung vorgegebenen Dosis des Bindemittels in die Matrizenkammer.

Weiterhin kann das Verfahren die folgenden Schritte aufweisen:
g) Verstellen des Magazins relativ zu der Matrizenkammer, bis das Magazin eine Stellposition einnimmt, in der ein linearverstellbarer Stempel des Magazins, der an einem freien Ende eine Stempelmatrize aufweist, einer Öffnung der Matrizenkammer zugewandt ist;
h) Verlagern des Stempels aus einer Freigabeposition, in der der Stempel mit seiner Stempelmatrize außer Eingriff mit der Matrizenkammer steht, in eine ausgefahrene Stellung, in der der Stempel mit seiner Stempelmatrize über die Öffnung in die Matrizenkammer hineinragt, wobei der mindestens eine in die Matrizenkammer dosierte Wirkstoff und gegebenenfalls ein Bindemittel verdichtet werden.

Ebenso kann das Verfahren weiterhin die Schritte aufweisen:
i) Verstellen des Magazins relativ zu der Matrizenkammer, bis das Magazin eine Stellposition einnimmt, in der ein Trägermaterialbehälter des Magazins, in dem ein Trägermaterial vorgehalten ist, mit seiner Düse der Matrizenkammer und/oder einer Grundplatte zugewandt ist, die die Matrize einer Tablettenverpackung aufweist; und
j) Dosieren des Trägermaterials in die Matrizenkammer und/oder die Matrize der Grundplatte.

Das Dosieren des Trägermaterials in die Matrizenkammer kann das Ausbilden einer Wirkstoffkapsel aufweisen. Das Dosieren des Trägermaterials in die Matrize der Grundplatte kann das Ausbilden eine Tablettenverpackung aufweisen.

Das beschriebene Verfahren kann dazu verwendet werden, eine kindersichere Blisterverpackung bzw. eine Rundumverpackung für einen Wirkstoff bzw. eine Wirkstofftablette herzustellen. Insbesondere ermöglicht es das Verfahren, dass die Herstellung der Tablette, die Herstellung der Verpackung sowie eine gegebenenfalls erforderliche Beschriftung der Verpackung und eventuell die Hinzufügung eines Datenträgers zu der Verpackung zur Bereitstellung von Inhaltsangaben und dergleichen sämtlich in einem einzigen Arbeitsgang bereitgestellt werden können.

Die zuvor beschriebene Vorrichtung sowie das Verfahren zur Tablettierung von Wirkstoffen können sich die aus dem Stand der Technik bekannten Prinzipien der additiven Fertigung, beispielsweise des 3D-Drucks, zunutze machen. Damit sind sämtliche Fertigungsverfahren gemeint, bei denen Festkörper Schicht für Schicht aufgetragen und so in der Folge dreidimensionale Gegenstände erzeugt werden können. Der schichtweise Aufbau erfolgt in der Regel computergesteuert unter Verwendung mindestens eines flüssigen oder festen Werkstoffs. Die Wirkstoffbehälter können in der Art von Druckerpatronen ausgebildet sein und jeweils eine Dosiereinrichtung aufweisen, die individuell ansteuerbar ist. Auf diese Weise wird sichergestellt, dass ein Kontakt der unterschiedlichen Wirkstoffe in der Vorrichtung ausgeschlossen ist. Die Düsen der Wirkstoffbehälter können beispielsweise analog zu Einmalkanülen austauschbar sein und nach jeder Verwendung der erfindungsgemäßen Vorrichtung beziehungsweise Anwendung des erfindungsgemäßen Verfahrens ausgewechselt werden.

Vorteilhafterweise werden die aufgebrachten Wirkstoffdosen beziehungsweise Wirkstoffschichten nach dem Dosieren sämtlicher Einzeldosen beziehungsweise Einzelschichten kompaktiert beziehungsweise verdichtet. Dies kann analog zu dem aus dem Stand der Technik bekannten Verpressen unter Verwendung eines Stempels und einer Matrize erfolgen. Die Matrize wird dabei von der Matrizenkammer gebildet. Der Stempel kann in dem Magazin vorgehalten sein und für das Verdichten beziehungsweise Kompaktieren kann das Magazin in eine Stellposition gebracht werden, in welcher der Stempel mit der Matrizenkammer fluchtet, so dass der Stempel beispielsweise durch Linearverstellung aus dem Magazin ausgefahren und in die Matrizenkammer eingeführt werden kann. Vorteilhafterweise ist auch eine Unterseite der Matrizenkammer als ein beweglicher Stempel ausgebildet, so dass beim Kompaktieren beziehungsweise Verdichten der dosierten Wirkstoffe in der Matrizenkammer die beiden Stempel einander angenähert werden, wobei eine homogene Verdichtung erreicht wird. Bekannte automatische Vorrichtungen zum Kompaktieren von Tabletten besitzen vertikal ausgerichtet übereinander angeordnet einen Unterstempel, der in einer Matrize läuft und einen Oberstempel, der in die Matrize nur zur Pressung eingeführt wird. Dem gegenüber kann bei der erfindungsgemäßen Vorrichtung vorgesehen sein, dass der Tablettenrohling direkt in die Matrizenkammer dosiert beziehungsweise gedruckt wird und gegebenenfalls für die weitere Verdichtung lediglich ein Oberstempel, der in dem Magazin vorgehalten ist, in die Matrize eingeführt wird. Damit hängen alleine von dem Oberstempel und seinem Pressdruck die Dicke, die Festigkeit und der Pressglanz der Tablette ab.

Die Einführtiefe und die Druckstärke des Oberstempels in die Matrizenkammer und die Druckstärke lassen sich mit der Formgebung des in der Matrizenkammer abgeschiedenen beziehungsweise darin dosierten Rohlings regulieren. Der Unterstempel kann dabei in der Matrize angeordnet sein und den Füllraum der Matrizenkammer nach unten begrenzen. Während des Verdichtungsvorgangs bildet er das Gegenlager für den in dem Magazin vorgehaltenen Oberstempel. Nach dem Abschluss des Pressvorgangs wird der Unterstempel nach oben geführt, wodurch die Tablette auf den Matrizenrand verlagert wird, wo sie beiseite geschoben werden kann. In einem optionalen nächsten Arbeitstakt kann der tablettierte Wirkstoff mit einem Wirkstoff getränkt oder mit einer Schutz- oder Funktionsschicht überzogen werden.

Die Wirkstoffbehälter können als austauschbare Kartuschen ausgebildet sein. Bei einer Ausführungsform der Erfindung kann in einem der Wirkstoffbehälter beziehungsweise in einer der Kartuschen ein Trägermaterial für die Herstellung einer Tablettenverpackung vorgehalten sein, beispielsweise für die Ausbildung einer Verblisterung, wozu beispielsweise ein biologisch abbaubarer Werkstoff vorgesehen sein kann, beispielsweise Silikon.

Das erfindungsgemäße Verfahren kann unter Reinraumverhältnissen durchgeführt werden, wozu die erfindungsgemäße Vorrichtung ein Umluftsystem mit HEPA-Filtern aufweisen kann. Die Sterilisierbarkeit der Vorrichtung kann durch ein UV- oder Ozonsystem oder durch ein sonstiges System sichergestellt sein. Die erfindungsgemäße Vorrichtung und das Verfahren können dazu verwendet werden, individuelle Zusammensetzungen von Wirkstoffen und Formgebungen von Tabletten zu steuern. Unter Verwendung patientenbezogener Daten können Tabletten innerhalb weniger Minuten kostengünstig auch in kleinen Mengen hergestellt werden.

Weitere Einzelheiten der Erfindung werden anhand der nachstehenden Figuren erläutert. Dabei zeigt:
- Figur 1: in perspektivischer und schematischer Darstellung eine beispielhafte Ausführungsform einer erfindungsgemäßen Vorrichtung;
- Figur 2: im Querschnitt und in schematischer Darstellung eine weitere Ausführungsform einer erfindungsgemäßen Vorrichtung;
- Figur 3: im Querschnitt und in schematischer Darstellung ein Detail einer weiteren Ausführungsform einer erfindungsgemäßen Vorrichtung;
- Figur 4: in perspektivischer und schematischer Darstellung eine Grundplatte gemäß einer Ausführungsform der Erfindung; und
- Figur 5: im Querschnitt und in schematischer Darstellung eine Ausführungsform einer Tablettenverpackung.

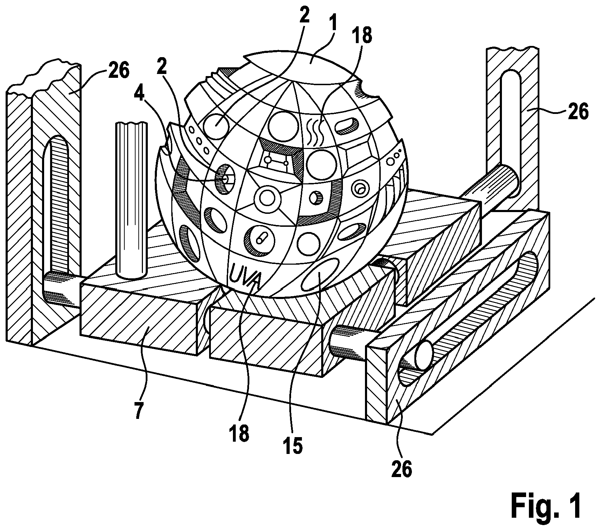

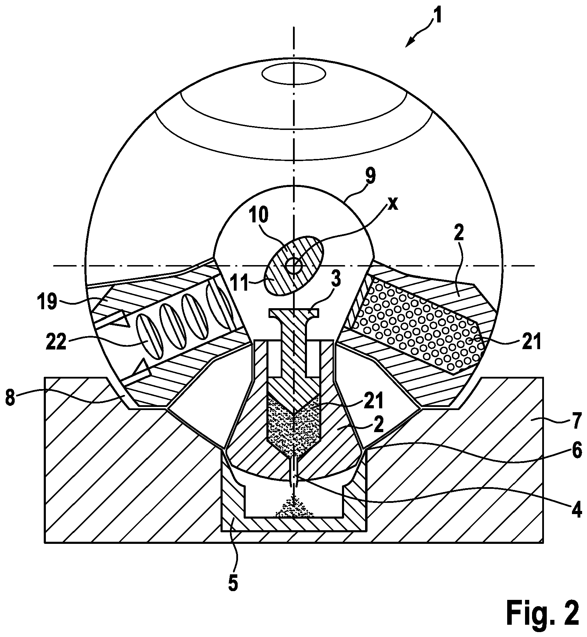

Die Figuren 1 und 2 zeigen jeweils beispielhafte Ausführungsformen einer erfindungsgemäßen Vorrichtung für die Tablettierung einer Wirkstoffzusammensetzung. Die Merkmale der beide n gezeigten Ausführungsformen können auch in einer einzigen Ausführungsform umgesetzt werden.

Die Einzelwirkstoffe der Wirkstoffzusammensetzung können in dem Magazin 1 der Vorrichtung voneinander separiert, insbesondere fluidisch voneinander getrennt, beispielsweise als pulverförmige, flüssige, pastöse, verkapselte oder granulare Wirkstoffe in jeweils einem Wirkstoffbehälter 2 bereitgestellt sein. Demgemäß weist das Magazin 1 eine Vielzahl Wirkstoffbehälter 2 auf, in denen jeweils ein Wirkstoff oder ein definiertes Wirkstoffgemisch vorgehalten ist, wobei jeder der Wirkstoffbehälter 2 eine separat ansteuerbare Dosiereinrichtung 3 aufweist. Die Wirkstoffbehälter 2 können weiterhin eine Düse 4 für den Wirkstoffauslass aufweisen.

Das Magazin 1 ist kugelförmig ausgebildet, so dass sich die Wirkstoffbehälter 2 in Radialrichtung des Magazins 1 erstrecken und mit ihrer jeweiligen Düse 4 in eine Kugeloberfläche des Magazins 1 münden. Das kugelförmige Magazin 1 kann zwischen einer Vielzahl Stellpositionen relativ zu mindestens einer Matrizenkammer 5 in der Grundplatte 7 verstellbar sein, wobei in jeder der Stellpositionen mindestens einer der Wirkstoffbehälter 2, insbesondere die jeweilige Düse 4 des betreffenden Wirkstoffbehälters 2 einer Öffnung 6 der Matrizenkammer 5 zugewandt ist, so dass bei einer Betätigung der Dosiereinrichtung 3 der in dem jeweiligen Wirkstoffbehälter 2 vorgehaltene Wirkstoff in die Matrizenkammer 5 dosiert werden kann.

Die Dosiereinrichtung 3 kann geeignete Mittel zur prozesssicheren Abgabe einer vorgegebenen Dosis in die Kammer 5 aufweisen, beispielsweise einen Durchflussmengenmesser, einen Gewichtsensor oder dergleichen.

Neben der Vielzahl Wirkstoffbehälter 2 kann das Magazin 1 weitere funktionale Elemente aufweisen, die über die Kugeloberfläche des Magazins 1 mit der Matrizenkammer 5 beziehungsweise einer darin vorgehaltenen Wirkstoffzusammensetzung in Kontakt treten können. Beispielsweise kann eines der funktionalen Elemente ein Mittel zum Verdichten der Wirkstoffzusammensetzung in der Matrizenkammer 5 sein, beispielsweise ein Stempel mit einer Matrize. Darüber hinaus kann das Magazin 1 mindestens einen Trägermaterialbehälter 19 aufweisen, in dem mindestens ein Trägermaterial 22 vorgehalten ist, welches bedarfsweise in die Matrizenkammer 5 appliziert werden kann, beispielsweise zur Ausbildung einer die Wirkstoffzusammensetzung beinhaltenden Wirkstoffkapsel.

Wie der Figur 2 weiter zu entnehmen ist, kann die mindestens eine Matrizenkammer 5, die in der Grundplatte 7 aufgenommen ist, in einer konkaven Ausnehmung 8 der Grundplatte 7 angeordnet sein, vorzugsweise an einer tiefsten Stelle der Ausnehmung 8, wobei das kugelförmige Magazin 1 in der konkaven Ausnehmung 8 gehalten ist. Das kugelförmige Magazin 1 kann zumindest teilweise formschlüssig in der Ausnehmung 8 aufgenommen sein, so dass das Magazin 1 in der Ausnehmung 8 geführt wird, wenn es seine Ausrichtung ändert.

Die Figur 2 zeigt weiterhin, dass das kugelförmige Magazin 1 in seinem Zentrum eine Kavität 9 aufweist, in welcher ein Betätigungselement 10 freigängig aufgenommen ist. Das Betätigungselement 10 kann insbesondere als drehbar gelagerter Nocken ausgebildet sein, wobei sich eine Drehachse x des Betätigungselements 10 außerhalb des Schwerpunkts des Betätigungselements 10 befindet. Da das Betätigungselement 10 freigängig rotierend in der Kavität 9 aufgenommen ist, kann es sich unabhängig von der Ausrichtung des Magazins 1 in Bezug auf die Grundplatte 7 stets mit seinem Schwerpunkt an einer tiefsten Stelle befinden und beispielsweise in Eingriff mit einer Dosiereinrichtung 3 gebracht werden, die in einer untersten Stelle des kugelförmigen Magazins 1 in die Flucht mit der Matrizenkammer 5 gebracht wird. Dadurch kann erreicht werden, dass mit dem Verdrehen des Magazins 1, wobei ein bestimmter Wirkstoffbehälter 2, in welchem ein bestimmter Wirkstoff 21 vorgehalten ist, in die unterste Stellung gebracht wird, die Dosiereinrichtung 3 des betreffenden Wirkstoffbehälters 2 aufgrund des schwerkraftgetriebenen Betätigungselements 10 betätigt wird.

Bei der Ausführungsform gemäß Figur 2 ist die Dosiereinrichtung nach Art eines Stempels ausgebildet, welcher bei der Verlagerung des Magazins 1 in der zuvor beschriebenen Weise von dem Betätigungselement weiter in den Wirkstoffbehälter 2 eingetrieben wird, wodurch der in dem Behälter 2 vorbehaltene Wirkstoff 21 über die in die Matrizenkammer 5 mündende Düse 4 des Wirkstoffbehälters 2 in die Matrizenkammer 5 hinein dosiert wird.

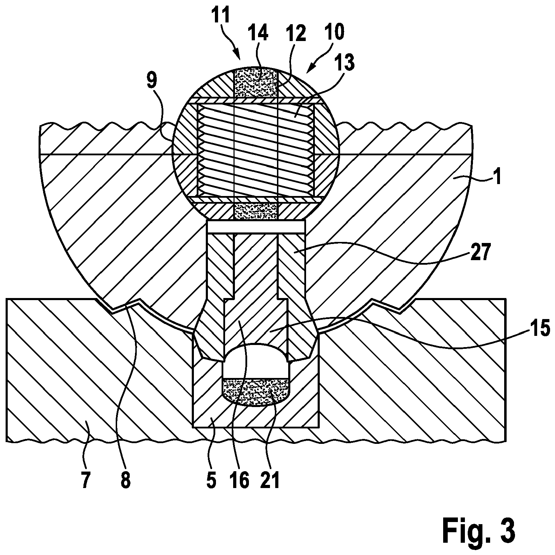

Die Figur 3 zeigt eine weitere Ausführungsform eines erfindungsgemäßen Magazins 1, wobei das Magazin 1 derart in Bezug auf die Matrizenkammer 5 in der Grundplatte 7 ausgerichtet ist, dass ein Stempel 15 mit einer endseitig angeordneten Stempelmatrize 16 mit der Matrizenkammer 5 in Eingriff gebracht werden kann, beispielweise um eine Verdichtung, Kompaktierung, Formgebung oder anderweitige mechanische Verfestigung des in der Matrizenkammer 5 enthaltenen Wirkstoffs 21 zu erzielen. Der Stempel 15 kann beispielsweise der weiteren Tablettierung des Wirkstoffs 21 dienen. Das Stempeln des Wirkstoffs 21 mit dem Stempel 15 ist jedoch erfindungsgemäß lediglich eine Variante zur Tablettierung des Wirkstoffs 21. Wie bereits mit Bezug auf die Figuren 1 und 2 beschrieben worden ist, kann auch durch das Dosieren des Wirkstoffs 21 bzw. der Wirkstoffe 21 in die Matrizenkammer 5 der Wirkstoff 21 die Kontur der Matrizenkammer 5 annehmen und insofern tablettiert werden.

Für die Verfestigung des Wirkstoffs in der Matrizenkammer, sei es unter Verwendung des Stempels 15 zur Kompaktierung oder eines weiteren mechanischen Hilfsmittels, kann dem Wirkstoff 21 ein Bindemittel zugesetzt sein, wie es grundsätzlich aus dem Stand der Technik bekannt ist. Das Bindemittel kann in einem separaten Wirkstoffbehälter 2 des Magazins 1 vorgehalten und in einem separaten Dosierschritt der Wirkstoffzusammensetzung 21 in der Matrizenkammer 5 zugesetzt werden oder bereits den Wirkstoffen 21 oder zumindest einem Teil der Wirkstoffe 21 in den Wirkstoffbehältern 2 des Magazins 1 beigemengt sein.

Die Figur 3 zeigt darüber hinaus eine weitere Ausführungsform eines Betätigungselements 10, das in der Kavität 9 im Zentrum des kugelförmigen Magazins 1 angeordnet ist. Bei dieser Ausführungsform ist abweichend von der Ausführungsform gemäß Figur 2 das Betätigungselement 10 ebenfalls kugelförmig ausgebildet und im Wesentlichen formschlüssig, jedoch wiederum frei beweglich in der Kavität 9 aufgenommen. Das Betätigungselement 10 weist ein linear verstellbares Stellglied 11 auf, das in der Darstellung gemäß Figur 3 in Vertikalrichtung zwischen einer Betätigungsposition und einer Freigabeposition verstellbar ist.

Das Betätigungselement 10 weist einen sich durch sein Zentrum erstreckenden Durchlass 12 auf, in dem eine Magnetspule 13 mit einem sich entlang der Spulenachse beweglichen Spulenkern 14 befindet. Der Spulenkern 14 ist stabförmig ausgebildet und erstreckt sich durch das Zentrum sowohl des Magazins 1 als auch des Betätigungselements 10. Der stabförmige Spulenkern 14 kann beispielsweise ein Permanentmagnet sein, welcher durch Strombeaufschlagung der Spule entlang der Spulenachse verlagert werden kann. In Figur 3 ist der Spulenkern 14 in seiner Freigabeposition gezeigt, in welcher der Spulenkern 14 nicht mit dem Stempel 15 in Eingriff steht. Durch Strombeaufschlagung kann der Spulenkern 14 aus der in Figur 3 gezeigten Stellung in eine Betätigungsstellung verlagert werden, in welcher er gegenüber der Freigabeposition vertikal nach unten verlagert ist und mit dem Stempel 15 in Eingriff steht. Insbesondere kann der Spulenkern dazu in die Linearführung 27 des Stempels 15 eintreten, wodurch der Stempel 15 mit seiner endseitigen Matrize 16 weiter in die Matrizenkammer 5 eingetrieben wird, um den in der Matrizenkammer 5 befindlichen Wirkstoff 21 beziehungsweise ein Wirkstoffgemisch, welches gegebenenfalls neben mindestens einem Wirkstoff auch ein Bindemittel aufweist, zu tablettieren. Anschließend kann durch Umkehr des Stromflusses der Spulenkern 14 wieder in die in Figur 3 gezeigte Freigabeposition überführt werden.

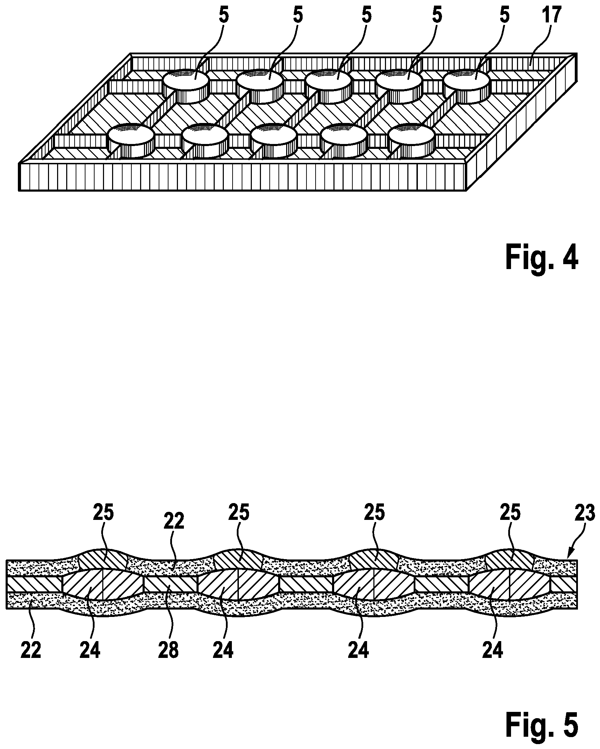

Die Figur 4 zeigt eine beispielhafte Ausführungsform einer Matrize 17, wie sie für die Ausbildung einer Vielzahl von Tabletten vorgesehen ist und gleichzeitig einer Tablettenverpackung, in welcher die erzeugten Tabletten verpackt sind. Dazu weist die Matrize 17 eine Mehrzahl in einem regelmäßigen Raster angeordnete Matrizenkammern 5 auf, so dass neben der Produktion der Tabletten in der Matrizenkammern 5 mit Hilfe der erfindungsgemäßen Vorrichtung, beispielsweise einer der in den Figuren 1 bis 3 gezeigten Vorrichtung, zusätzlich zu den Tabletten 24 auch Trägermaterial in die Matrize 17 eingebracht werden kann, so dass verpackte Tabletten 24 hergestellt werden können.

Die Figur 5 zeigt einen Querschnitt durch eine beispielhafte Ausführungsform einer Tablettenverpackung 23 mit darin aufgenommenen Tabletten 24, die unter Zuhilfenahme beispielsweise der in den Figuren 1 bis 4 gezeigten Vorrichtung hergestellt worden ist. Demgemäß weist die Tablettenverpackung 23 im Querschnitt eine Schichtabfolge auf, bestehend aus einer unteren Trägermaterialschicht 22, auf der neben den Tabletten 24 weiterhin die Tabletten 24 voneinander separierende Trennelemente 28 angeordnet sind. Über den Tabletten 24 und den Trennelementen 28 ist eine weitere Trägermaterialschicht 22 ausgebildet mit darin eingebetteten Verschlüssen 25 oberhalb jeder Tablette 24.

Sämtliche der in Figur 5 gezeigten Elemente 22, 23, 24, 25, 28 können mit Hilfe der erfindungsgemäßen Vorrichtung in einem entsprechenden Druckverfahren hergestellt werden. Beispielsweise kann mit der in Figur 1 gezeigten Vorrichtung unter Verwendung der in Figur 4 gezeigten Matrize in einem ersten Verfahrensschritt eine durchgängige, das heißt ununterbrochene Trägermaterialschicht 22 in die Matrize 17 eingebracht werden, das Trägermaterial 22 kann beispielsweise ein Kunststoffmaterial sein. Nachdem die Trägermaterialschicht 22 in die Matrize 17 eingebracht und gegebenenfalls gehärtet worden ist, können in einem nächsten Schritt mit derselben Vorrichtung 1 patientenbedarfsweise die Tabletten 24 in den Matrizenkammern 5 und oberhalb der in diesen bereits ausgehärteten Trägermaterialschicht 22 ausgeformt werden, wie dies beispielsweise mit Bezug auf die vorangegangenen Figuren erläutert wurde. Analog zu der Trägermaterialschicht 22 können zwischen den Tabletten 24 die Trennelemente 28 abgeschieden werden. Anschließend werden oberhalb von den Tabletten 24 die Verschlüsse 25 ausgebildet und in einem letzten Schritt die Matrize 17 um die Verschlüsse 25 herum vollständig mit Trägermaterial 22 aufgefüllt. Sämtliche Applikationen der zuvor genannten Wirkstoffe und sonstigen Materialien, einschließlich der Trägermaterialien 22, der Materialien für die Verschlüsse 25 sowie die Trennelemente 28 können mit Hilfe einer der in den Figuren 1 bis 3 gezeigten Vorrichtungen hergestellt werden.

Das Dosieren der Wirkstoffe und anderen Stoffe kann nach dem Prinzip des 3D-Druckens erfolgen. Die Erfindung kann somit gemäß einer Ausführungsform als eine automatische Vorrichtungen zur Herstellung von Tabletten basierend auf dem Prinzip des 3D-Drucks, auch bekannt unter den Bezeichnungen Additive Fertigung, Additive Manufacturing Generative Fertigung oder Rapid Technologien, realisiert sein.

Der schichtweise Aufbau kann computergesteuert aus einem oder mehreren flüssigen oder festen Wirkstoffen und mindestens einem Bindemittel erfolgen. Die Wirkstoffe und das Bindemittel sind dabei in als Druckerpatronen ausgebildeten Wirkstoffbehältern bevorratet, die jeweils eine Dosiereinrichtung aufweisen und individuell angesteuert werden können. Somit ist gesichert, dass ein Kontakt der Wirkstoffe untereinander ausgeschlossen ist.

Die Düsen an den Druckerpatronen können ähnlich wie Eimalkanülen austauchbar sein und werden nach jedem Druckvorgang ausgetauscht. Eine besondere Ausführungsform sieht vor, dass das Tablettiergut nach dem 3D-Druck mit derselben Vorrichtung mit Hilfe eines einzigen Pressvorganges zu einer Tablette verpresst wird. Hierzu können zwei bewegliche Stempel als Presswerkzeuge vorgesehen sein. Bekannte automatische Vorrichtungen zur Herstellung von Tabletten besitzen vertikal ausgerichtet einen Unterstempel, der in einer Matrize läuft und einen Oberstempel, der in die Matrize nur zur Pressung eingeführt wird. Bei einer Ausführungsform der Erfindung wird der Tablettenrohling direkt in der Matrizenkammer gedruckt und danach gleitet der Oberstempel in die Matrize, und presst die Tablette. Von seiner Geometrie und seinem Pressdruck hängen Dicke, Festigkeit und Pressglanz der Tablette ab. Die Einführungstiefe und die Druckstärke lassen sich mit der Formgebung des 3D-Rohlings regulieren. Der Unterstempel kann dabei innerhalb der Matrize angeordnet sein. Er begrenzt den Füllraum nach unten. Während des Pressvorgangs bildet er in der Regel das Gegenlager. Nach Abschluss der Pressung kann er nach oben geführt werden und bringt dadurch die Tablette auf den Matrizenrand, wo sie beiseitegeschoben werden kann. In einem nächsten Takt kann der Rohling, wenn er aus saugfähigen Material besteht, mit Wirkstoffen getränkt oder mit einer Schutz- oder Funktionsschicht überzogen werden. Die Schicht kann folgend Eigenschaften haben.

Das Magazin kann mehrere auswechselbare Kartuschen besitzen und es ist bei einer besonderen Auführungsform der Erfindung vorgesehen, mit demselben Magazin und derselben Vorrichtung die sonst übliche Verblisterung von Tabletten zu ersetzen. Hierfür kann ein biologisch abbaubarer Werkstoff vorgesehen sein, beispielsweise Silikon. Der komplette Vorgang kann in einem Bauraum der Vorrichtung durchgeführt werden, der hermetisch abgeriegelt ist. Für Reinraumverhältnisse sorgt ein Umluftsystem mit HEPA-Filtern. Die Sterilisierbarkeit der Vorrichtung ist durch eine UV- oder Ozonbehandlung gegeben, oder durch andere geeignete Systeme.

Der größte Vorteil der Erfindung liegt in der individuell steuerbaren Zusammensetzung der Wirkstoffe und Formgebung der Tabletten. Durch die Steuerbarkeit der Vorrichtung und die Einspeisung von patientenbezogenen Daten können Tabletten innerhalb weniger Minuten kostengünstig auch in kleinen Mengen hergestellt werden.

Die Grundplatte kann zusätzlich zu der mindestens einen Matrizenkammer eine Pressvorrichtungen mit einer weiteren Matrize in Form einer Tablette aufweisen und/oder Haltevorrichtungen für Tablettenrohlinge, Karpulen, Ampullen, oder Spritzenkörpern. Ebenso können die Wirkstoffbehälter des Magazins als Druckerpatronen oder Kartuschen ausgebildet sein, die beispielsweise nicht nur Wirkstoffe enthalten und dosieren können, sondern auch dazu eingerichtet sein können, diese auch in eine Matrize mit bis zu 80 kN/cm³ pressen zu können, etwa indem sie einen Stempel mit einer endseitigen Matrize aufweisen.

Eine Ausführungsform der Erfindung betrifft eine 3D- Medikamenten-Dosiervorrichtung, die aus einer auswechselbaren Einheit und einer festinstallierten Einheit besteht. Die auswechselbare Einheit weist Wirkstoffe und ggf. sonstige Substanzen in Form von Pulver, Partikeln, Fluid oder dergleichen in jeweils einem geeigneten Reservoir auf, das in Form einer unter Druck stehenden Druckerpatrone zur Aufnahme des Wirkstoffs oder der sonstigen Substanz bereitgestellt sein kann.

Das Magazin kann abweichend von den bekannten, bauüblichen Tablettenmaschinen nicht feststehend als Presseinrichtungen gestaltet sein, sondern vertikal, und horizontal ausgerichtet und in X- Y- und Z-Richtung beweglich sein. Durch diese Maßnahme können auch 3D-Formlinge aus Trägermaterial für Wirkstoffe ohne Pressformen hergestellt werden. Die Rohlinge können auch mit Presswerkzeugen in Form gebracht werden. Die Dosiereinnrichtungen können auch nur zum Füllen der Presswerkzeuge oder Hohlkörper, die im gleichen System vorher gedruckt wurden, dienen. Die Hohlkörper können aus saugfähigen Schwamm/Schaum-Material bestehen, das sehr saugfähig ist, z.B. Kieselsäure, Hyaluron oder Substanzen die gerne eine Verbindung mit den Wirkstoffen eingehen.

Die Dosiereinrichtung kann einen Sensor zum Erfassen der Durchflussmenge eines flüssigen Wirkstoffs oder Bindemittels, einen mit einem Fließwiderstand versehenen, fluidmäßig mit dem Wirkstoffreservoir verbundenen Fluidkanal und mindestens eine mit dem Fluidkanal verbundene Düse aufweisen.

An einer Kugeloberfläche des Magazin können konkave Matrizen angebracht sein, die Wirkstoffe in eine gewünschte Form bringen können. Besonders vorteilhaft hat sich herausgestellt, das kugelförmige Magazin in einer teil- oder halbkugelartigen Halterung frei beweglich zu lagern. Die Halterung wiederum kann in X-, Y- und Z-Richtung beweglich sein. Die Halterung kann mindestens vier rotierbare Antriebe aufweisen, die das Magazin in alle Richtung rotieren können. Hierdurch ist es möglich alle in der Kugel befindlichen Kartuschen oder Werkzeuge in jeder Ebene im Raum zu nutzen. Kontaktflächen an den Oberflächen und zu aktivierende Elektromagnete garantieren eine sichere Justierung.

Ein in dem Wirkstoffbehälter enthaltener flüssiger Wirkstoffwird kann vorzugsweise mithilfe eines Konstantdruckgebers mit einem Druck beaufschlagt. Die Vorrichtung kann zumindest einen Drucksensor aufweisen, um den Druck des flüssigen Medikaments vor einem Flusswiderstand des Wirkstoffbehälters zu erfassen. Ferner kann ein weiterer Drucksensor nach dem Flusswiderstand vorgesehen sein, wobei eine Steuereinrichtung dazu eingerichtet ist, die Flussrate eines flüssigen Wirkstoffs zu steuern.

Das Dosiersystem kann nach dem Überdruckprinzip arbeiten und verwendet vorzugsweise einen mikromechanisch gefertigten optischen Sensor und bietet somit die Möglichkeiten, zur externen Beeinflussung der Dosierrate über die Steuervorrichtung zur Kompensation von eventuellen Defekten an der gedruckten Tablette.

Das Dosiersystem kann ein Quetschventil aufweisen, das von der Steuereinrichtung getaktet dazu angesteuert wird, um einen Durchfluss durch einen Schlauch des Dosiersystems zu ermöglichen oder zu verhindern. Dieses Quetschventil nimmt den Schlauch von außen in Eingriff und quetscht ihn zusammen, um einen Durchfluss des flüssigen Medikaments zu verhindern. Dadurch kommt das Ventil nicht mit dem Medikament in Berührung. Somit kann das Ventil ein Teil der dauerhaften Vorrichtung sein.

### Bezugszeichenliste

- 1: Magazin
- 2: Wirkstoffbehälter
- 3: Dosiereinrichtung
- 4: Düse
- 5: Matrizenkammer
- 6: Öffnung
- 7: Grundplatte
- 8: Ausnehmung
- 9: Kavität
- 10: Betätigungselement
- 11: Stellglied
- 12: Durchlass
- 13: Magnetspule
- 14: Spulenkern
- 15: Stempel
- 16: Stempelmatrize
- 17: Matrize
- 18: Verarbeitungseinrichtung
- 19: Trägermaterialbehälter
- 20: Bindemittelvorratsbehälter
- 21: Wirkstoff
- 22: Trägermaterial
- 23: Tablettenverpackung
- 24: Tablette
- 25: Verschluss
- 26: Kinematik
- 27: Linearführung
- 28: Trennelement
- X: Drehachse

## Patentansprüche

1. Vorrichtung für die Tablettierung einer pulverförmigen, flüssigen, pastösen, verkapselten oder granularen Wirkstoffzusammensetzung, mit mindestens einer Matrizenkammer (5) und einem Magazin (1), das eine Vielzahl Wirkstoffbehälter (2) mit jeweils einer einzeln ansteuerbaren Dosiereinrichtung (3) und einer Düse (4) für den Wirkstoffauslass aufweist, wobei das Magazin (1) zwischen einer Vielzahl Stellpositionen relativ zu der mindestens einen Matrizenkammer (5) verstellbar ist, wobei in jeder der Stellpositionen mindestens eine der Düsen (4) einer Öffnung (6) der Matrizenkammer (5) zugewandt ist, **dadurch gekennzeichnet, dass** das Magazin (1) kugelförmig ausgebildet ist, wobei sich die Wirkstoffbehälter (2) in Radialrichtung des Magazins (1) erstrecken und mit ihrer jeweiligen Düse (4) in eine Kugeloberfläche des Magazins (1) münden.

2. Vorrichtung nach Anspruch 1, bei der die mindestens eine Matrizenkammer (5) in einer Grundplatte (7) aufgenommenen ist, die eine konkave Ausnehmung (8) aufweist, in der das kugelförmige Magazin (1) aufgenommen ist.

3. Vorrichtung nach Anspruch 2, bei der an einer tiefsten Stelle der Ausnehmung (8) die mindestens eine Matrizenkammer (5) angeordnet ist.

4. Vorrichtung nach Anspruch 2 oder 3, bei der das Magazin (1) um mindestens eine sich parallel zu der Grundplatte (7) erstreckende Achse relativ zu der Grundplatte (7) verstellbar ist.

5. Vorrichtung nach einem der vorangegangenen Ansprüche, bei der das kugelförmige Magazin (1) in seinem Zentrum eine Kavität (9) aufweist, in der ein Betätigungselement (10) frei beweglich aufgenommen ist, wobei das Betätigungselement (10) ein Stellglied (11) aufweist, das zwischen einer Bestätigungsposition und einer Freigabeposition verstellbar ist.

6. Vorrichtung nach Anspruch 5, bei der entweder das Betätigungselement (10) kugelförmig ausgebildet und formschlüssig in der Kavität (9) aufgenommen ist, oder bei der das Betätigungselement (10) eine drehbar gelagerter Nocken mit außerhalb der Drehachse (x) liegendem Schwerpunkt ist, bei dem das Stellglied (11) ein Nockenvorsprung ist.

7. Vorrichtung nach Anspruch 5 oder 6, bei der das Betätigungselement (10) einen sich durch sein Zentrum erstreckenden Durchlass (12) aufweist, in dem eine Magnetspule (13) mit einem entlang der Spulenachse beweglichen Spulenkern (14) angeordnet ist.

8. Vorrichtung nach einem der vorangegangenen Ansprüche, bei der das Magazin (1) einen linearverstellbaren Stempel (15) aufweist, der in einer der Stellpositionen oder in einer weiteren Stellposition des Magazins (1) relativ zu der Matrizenkammer (5) der Öffnung (6) der Matrizenkammer (5) zugewandt ist.

9. Vorrichtung nach Anspruch 8, bei der der Stempel (15) an einem freien Ende eine Stempelmatrize (16) aufweist, mit der der Stempel (15) in einer ausgefahrenen Stellung über die Öffnung (6) in die Matrizenkammer (5) hineinragt, und mit der der Stempel (15) in einer Freigabeposition außer Eingriff mit der Matrizenkammer (5) steht.

10. Vorrichtung nach einem der vorangegangenen Ansprüche, bei der mindestens einer der Wirkstoffbehälter (2) eine Verarbeitungseinrichtung (18) für einen Wirkstoff aufweist.

11. Vorrichtung nach Anspruch 10, bei der die Verarbeitungseinrichtung (18) ein Heizelement zum Erhitzen und/oder ein Kühlelement zum Kühlen eines Wirkstoffs ist oder aufweist.

12. Vorrichtung nach einem der vorangegangenen Ansprüche, bei der das Magazin (1) zusätzlich zu der Vielzahl Wirkstoffbehälter (2) mindestens einen Trägermaterialbehälter (19) mit einer einzeln ansteuerbaren Dosiereinrichtung (3) und einer Düse (4) für den Trägermaterialauslass aufweist, wobei in einer weiteren Stellpositionen des Magazins (1) die Düse (4) des Trägermaterialbehälters (19) einer Öffnung (6) der Matrizenkammer (5) zugewandt ist.

13. Verfahren für die Tablettierung eines Wirkstoffs mit einer Vorrichtung nach einem der vorangegangenen Ansprüche, das die Schritte aufweist:
a. Vorgeben einer zu tablettierenden Wirkstoffzusammensetzung;
b. Verstellen des Magazins (1) relativ zu der Matrizenkammer, (5) bis das Magazin (1) eine Stellposition einnimmt, in der derjenige Wirkstoffbehälter (2) des Magazins (1) mit seiner Düse (4) der Matrizenkammer (5) zugewandt ist, in dem ein Wirkstoff der Wirkstoffzusammensetzung vorgehalten ist;
c. Dosieren einer durch die Wirkstoffzusammensetzung vorgegebenen Dosis des ersten Wirkstoffs in die Matrizenkammer (5); und
d. optional Wiederholen der Schritte b. und c. für jeden weiteren Wirkstoff der Wirkstoffzusammensetzung, wenn die Wirkstoffzusammensetzung mehr als einen Wirkstoff aufweist.

14. Verfahren nach Anspruch 13, das die weiteren Schritte aufweist:
e. Verstellen des Magazins (1) relativ zu der Matrizenkammer, (5) bis das Magazin (1) eine Stellposition einnimmt, in der ein linearverstellbarer Stempel (15) des Magazins (1), der an einem freien Ende eine Stempelmatrize (16) aufweist, einer Öffnung (6) der Matrizenkammer (5) zugewandt ist;
f. Verlagern des Stempels (15) aus einer Freigabeposition, in der der Stempel (15) mit seiner Stempelmatrize (16) außer Eingriff mit der Matrizenkammer (5) steht, in eine ausgefahrene Stellung, in der der Stempel (15) mit seiner Stempelmatrize (16) über die Öffnung (6) in die Matrizenkammer (5) hineinragt, wobei der mindestens eine in die Matrizenkammer (5) dosierte Wirkstoff und gegebenenfalls ein Bindemittel verdichtet werden.

15. Verfahren nach Anspruch 13 oder 14, das die weiteren Schritte aufweist:
g. Verstellen des Magazins (1) relativ zu der Matrizenkammer, (5) bis das Magazin (1) eine Stellposition einnimmt, in der ein Trägermaterialbehälter (19) des Magazins (1), in dem ein Trägermaterial vorgehalten ist, mit seiner Düse (4) der Matrizenkammer (5) zugewandt ist; und
h. Dosieren des Trägermaterials in die Matrizenkammer (5).

## Claims

1. A device for tableting a pulverulent, liquid, pasty, encapsulated or granular active ingredient composition, comprising at least one die chamber (5) and a magazine (1) which has a plurality of active ingredient containers (2) each having an individually controllable metering device (3) and a nozzle (4) for the active ingredient outlet, wherein the magazine (1) is adjustable between a plurality of adjustment positions relative to the at least one die chamber (5), wherein in each of the adjustment positions at least one of the nozzles (4) faces an opening (6) of the die chamber (5), **characterized in that** the magazine (1) is spherical, wherein the active ingredient containers (2) extend in the radial direction of the magazine (1) and open with their respective nozzle (4) into a spherical surface of the magazine (1).

2. The device according to claim 1, wherein the at least one die chamber (5) is received in a base plate (7) which has a concave recess (8) in which the spherical magazine (1) is received.

3. The device according to claim 2, wherein the at least one die chamber (5) is arranged at a lowest point of the recess (8).

4. The device according to claim 2 or 3, wherein the magazine (1) is adjustable relative to the base plate (7) about at least one axis extending parallel to the base plate (7).

5. The device according to one of the preceding claims, wherein the spherical magazine (1) has a cavity (9) in its center in which an actuating element (10) is received in a freely movable manner, wherein the actuating element (10) has an actuating element (11) which is adjustable between a confirmation position and a release position.

6. The device according to claim 5, wherein either the actuating element (10) is formed in a spherical manner and is received in a form-fitting manner in the cavity (9), or wherein the actuating element (10) is a rotatably mounted cam with a center of gravity lying outside the axis of rotation (x), wherein the actuating element (11) is a cam projection.

7. The device according to claim 5 or 6, wherein the actuating element (10) has a passage (12) extending through its center in which a magnet coil (13) with a coil core (14) movable along the coil axis is arranged.

8. The device according to one of the preceding claims, wherein the magazine (1) has a linearly adjustable plunger (15) which faces the opening (6) of the die chamber (5) in one of the actuating positions or in a further actuating position of the magazine (1) relative to the die chamber (5).

9. The device according to claim 8, wherein the plunger (15) has a plunger die (16) at a free end, with which the plunger (15) projects into the die chamber (5) via the opening (6) in an extended position, and with which the plunger (15) is out of engagement with the die chamber (5) in a release position.

10. The device according to one of the preceding claims, wherein at least one of the active substance containers (2) has a processing device (18) for an active substance.

11. The device according to claim 10, wherein the processing device (18) is or has a heating element for heating and/or a cooling element for cooling an active substance.

12. The device according to one of the preceding claims, wherein the magazine (1) has, in addition to the plurality of active substance containers (2), at least one carrier material container (19) with an individually controllable metering device (3) and a nozzle (4) for the carrier material outlet, wherein the nozzle (4) of the carrier material container (19) faces an opening (6) of the die chamber (5) in a further setting position of the magazine (1).

13. A method for tableting an active substance with a device according to one of the preceding claims, which has the steps:
a. specifying an active substance composition to be tableted;
b. adjusting the magazine (1) relative to the die chamber (5) until the magazine (1) assumes an actuating position in which that active substance container (2) of the magazine (1) with its nozzle (4) faces the die chamber (5) in which an active substance of the active substance composition is stored;
c. dosing a dose of the first active substance specified by the active substance composition into the die chamber (5); and
d. optionally repeating steps b. and c. for each further active substance of the active substance composition if the active substance composition has more than one active substance.

14. The method according to claim 13, which has the further steps:
e. adjusting the magazine (1) relative to the die chamber (5) until the magazine (1) assumes an actuating position in which a linearly adjustable plunger (15) of the magazine (1), which has a plunger die (16) at a free end, faces an opening (6) of the die chamber (5);
f. displacing the plunger (15) from a release position, in which the plunger (15) is out of engagement with the die chamber (5) with its plunger die (16), into an extended position, in which the plunger (15) projects into the die chamber (5) via the opening (6) with its plunger die (16), wherein the at least one active substance dosed into the die chamber (5) and optionally a binder are compressed.

15. The method according to claim 13 or 14, which has the further steps:
g. adjusting the magazine (1) relative to the die chamber (5) until the magazine (1) assumes an actuating position in which a carrier material container (19) of the magazine (1) in which a carrier material is stored faces the die chamber (5) with its nozzle (4); and
h. dosing the carrier material into the die chamber (5).

## Revendications

1. Dispositif pour le pastillage d'une composition d'agents actifs pulvérulente, liquide, pâteuse, en capsulée ou granulaire, avec au moins une chambre de matrice (5) et un magasin (1), qui comprend une pluralité de récipients d'agents actifs (2) avec chacun un dispositif de dosage (3) pouvant être contrôlé individuellement et une buse (4) pour la sortie de l'agent actif, dans lequel le magasin (1) peut être réglé entre une pluralité de positions de réglage par rapport à l'au moins une chambre de matrice (5), dans lequel, dans chacune des positions de réglage, au moins une des buses (4) est orientée vers une ouverture (6) de la chambre de matrice (5), **caractérisé en ce que** le magasin (1) présente une forme sphérique, dans lequel les récipients d'agents actifs (2) s'étendent dans la direction radiale du magasin (1) et débouchent, avec leur buse (4) respective, dans une surface sphérique du magasin (1).

2. Dispositif selon la revendication 1, dans lequel l'au moins une chambre de matrice (5) est logée dans une plaque de base (7) qui présente un évidement concave (8) dans laquelle est logé le magasin sphérique (1).

3. Dispositif selon la revendication 2, dans lequel, au niveau de l'endroit le plus bas de l'évidement (8), est disposée l'au moins une chambre de matrice (5).

4. Dispositif selon la revendication 2 ou 3, dans lequel le magasin (1) peut être réglé par rapport à la plaque de base (7) autour d'au moins un axe s'étendant parallèlement à la plaque de base (7).

5. Dispositif selon l'une des revendications précédentes, dans lequel le magasin sphérique (1) présente, en son centre, une cavité (9) dans laquelle un élément d'actionnement (10) est logé de façon à pouvoir se déplacer librement, dans lequel l'élément d'actionnement (10) comprend un organe de réglage (11) qui peut être réglé entre une position d'actionnement et une position de libération.

6. Dispositif selon la revendication 5, dans lequel soit l'élément d'actionnement (10) présente une forme sphérique et est logé par complémentarité de forme dans la cavité (9) soit l'élément d'actionnement (10) est une came logée de manière rotative avec un centre de gravité se trouvant hors de l'axe de rotation (x), dans lequel l'organe de réglage (11) est une saillie de la came.

7. Dispositif selon la revendication 5 ou 6, dans lequel l'élément d'actionnement (10) comprend un passage (12) s'étendant à travers son centre, dans lequel est disposée une bobine magnétique (13) avec un noyau de bobine (14) mobile le long de l'axe de la bobine.

8. Dispositif selon l'une des revendications précédentes, dans lequel le magasin (1) comprend un poinçon réglable de manière linéaire (15) qui est orienté, dans une des positions de réglage ou dans une autre position de réglage du magasin (1) par rapport à la chambre de matrice (5), vers l'ouverture (6) de la chambre de matrice (5).

9. Dispositif selon la revendication 8, dans lequel le poinçon (15) comprend, au niveau de son extrémité libre, une matrice de poinçon (16) avec laquelle le poinçon (15), dans une position déployée, pénètre, par l'ouverture (6), dans la chambre de matrice (5) et avec laquelle le poinçon (15) est déboîté avec la chambre de matrice (5) dans une position de libération.

10. Dispositif selon l'une des revendications précédentes, dans lequel au moins un des récipients d'agents actifs (2) comprend un dispositif de traitement (18) pour un agent actif.

11. Dispositif selon la revendication 10, dans lequel le dispositif de traitement (18) est ou comprend un élément chauffant pour le chauffage et/ou un élément de refroidissement pour le refroidissement d'un agent actif.

12. Dispositif selon l'une des revendications précédentes, dans lequel le magasin (1) comprend, en plus de la pluralité de récipients d'agents actifs (2), au moins un récipient d'excipient (19) avec un dispositif de dosage (3) pouvant être contrôlé individuellement et une buse (4) pour la sortie de l'excipient, dans lequel, dans une autre position de réglage du magasin (1), la buse (4) du récipient d'excipient (19) est orientée vers une ouverture (6) de la chambre de matrice (5).

13. Procédé de pastillage d'un agent actif avec un dispositif selon l'une des revendications précédentes, qui comprend les étapes suivantes :
a. indication d'une composition d'agents actifs à pastiller ;
b. réglage du magasin (1) par rapport à la chambre de matrice (5) jusqu'à ce que le magasin (1) soit dans une position de réglage dans laquelle le récipient d'agent actif (2) du magasin (1), dans lequel est contenu un agent actif de la composition d'agents actifs, est orienté, avec sa buse (4), vers la chambre de matrice (5) ;
c. dosage d'une dose du premier agent actif prédéterminée par la composition d'agents actifs, dans la chambre de matrice (5) ; et
d. répétition optionnelle des étapes b. et c. pour chaque agent actif supplémentaire de la composition d'agents actifs, lorsque la composition d'agents actifs comprend plusieurs agents actifs.

14. Procédé selon la revendication 13, qui comprend les étapes supplémentaires suivantes :
e. réglage du magasin (1) par rapport à la chambre de matrice (5) jusqu'à ce que le magasin (1) soit dans une position de réglage dans laquelle un poinçon à déplacement linéaire (15) du magasin (1), qui comprend, au niveau d'une extrémité libre, une matrice de poinçon (16), est orienté vers une ouverture (6) de la chambre de matrice (5) ;
f. déplacement du poinçon (15) de la position de libération, dans laquelle le poinçon (15), avec sa matrice de poinçon (16), est déboîtée de la chambre de matrice (5), vers une position déployée dans laquelle le poinçon (15) pénètre, avec sa matrice de poinçon (16), par l'ouverture (6), dans la chambre de matrice (5), dans lequel l'au moins un agent actif dosé dans la chambre de matrice (5) et, le cas échéant un liant, sont comprimés.

15. Procédé selon la revendication 13 ou 14, qui comprend les étapes supplémentaires suivantes :
g. réglage du magasin (1) par rapport à la chambre de matrice (5) jusqu'à ce que le magasin (1) soit dans une position de réglage dans laquelle un récipient d'excipient (19) du magasin (1), dans lequel un excipient est contenu, est orienté, avec sa buse (4), vers la chambre de matrice (5) ; et
h. dosage de l'excipient dans la chambre de matrice (5).
